# EUROPÄISCHE PATENTSCHRIFT

(11) **EP 2 514 380 B1**
(45) Veröffentlichungstag und Bekanntmachung des Hinweises auf die Patenterteilung: **02.10.2013**
(21) Anmeldenummer: 11163501.7
(22) Anmeldetag: 21.04.2011
(51) Int. Cl.: A61B 18/12, A61B 18/00

(54) **Elektrochirurgische Einrichtung mit verbessertem Abschnitt**
Electrical surgical device with improved cutting
Appareil électrochirurgical avec dispositif de coupe améliorée

(43) Veröffentlichungstag der Anmeldung: 24.10.2012
(73) Patentinhaber: ERBE Elektromedizin GmbH, 72072 Tübingen (DE)
(72) Erfinder: Werner, Erich, 72827, Wannweil (DE)
(74) Vertreter: Rüger, Barthelt & Abel

(56) Entgegenhaltungen:
- EP-A1- 1 849 425
- EP-A1- 1 862 137
- WO-A1-2004/062516
- DE-A1- 2 504 280

## Beschreibung

Die Erfindung betrifft eine elektrochirurgische Einrichtung, die insbesondere ein Gerät zur Versorgung eines chirurgischen Instruments mit elektrischer Leistung umfasst.

Elektrochirurgische Einrichtungen oder Systeme sind seit längerem in Gebrauch. Zum Beispiel offenbart die EP 1 053 720 A1 ein elektrochirurgisches System, zu dem ein chirurgisches Instrument und ein Gerät gehören, das das elektrochirurgische Instrument mit HF-Leistung versorgt. Dazu dient ein HF-Generator, der die entsprechende HF-Spannung liefert. Der Generator wird zur Regulierung der an der Elektrode umgesetzten Leistung in schneller Folge ein- und ausgeschaltet, wobei über die Einstellung des Tastverhältnisses eine Leistungsregulierung möglich ist. Damit soll insbesondere eine Überhitzung der Elektrode vermieden werden.

Zur Erfassung der Elektrodentemperatur wird vorgeschlagen, die an der Elektrode anliegende Spannung auf Gleichanteile zu untersuchen, um einen thermoionischen Effekt, d.h. eine temperaturbedingte Elektronenemission zu erfassen, wenn die Elektrode zu heiß wird. Der angeschlossene Prozessor erfasst dies und reguliert das Tastverhältnis der HF-Spannung nach, um die gewünschte Leistungsreduzierung zu bewirken. Damit soll eine Elektrodenschädigung vermieden werden.

Aus der EP 1862137 sind ein System und ein Verfahren zur Gewebekoagulation bekannt. Bei der Gewebekoagulation wird elektrischer Strom in biologisches Gewebe eingeleitet, um dies zu erwärmen und zum Verkleben zu bringen. Hierbei werden verschiedene Phasen (Phase I und Phase II) unterschieden, die mit unterschiedlichen Gewebewiderständen einhergehen. Das beschriebene System reguliert den Energieeintrag in das Gewebe so, dass ein gewünschter Gewebeimpedanzverlauf erhalten wird.

Weiter ist aus der WO 2004/062516 A1 eine Einrichtung zum Schneiden von biologischem Gewebe mittels Plasmaskalpells bekannt. Bei dieser wird ein Plasmaskalpell über einen Hochfrequenzgenerator mit HF-Leistung versorgt, wobei die HF-Quelle getaktet wird, um eine gewünschte Ausgangsleistung zu erzielen. Bei Anschnittbeginn ist der Gewebewiderstand niedrig, so dass mit einem hohen Crestfaktor gearbeitet wird. Nach erfolgtem Anschnitt und Zünden des HF-Funkens, steigt der Lastwiderstand an, so dass der Crestfaktor zurückgeht und auch mit kontinuierlicher HF-Ausgangswelle gearbeitet werden kann.

Weiter ist es bspw. aus der US 2009/0209956 A1 bekannt, bei einem elektrochirurgischen Gerät den Scheitelfaktor des Elektrodenstroms zu überwachen, um daraus Rückschlüsse zu ziehen, ob sich die Elektrode im Anschnitt oder im Schneidbetrieb befindet. Im Anschnitt ist die Elektrode von ionenleitfähigem Wasser, z.B. NaCl-Lösung umgeben. Sie führt einen relativ hohen Strom. Dieser führt zur Bildung einer Dampfblase an der Elektrode, in der sich dann eine Plasmaentladung ausbilden kann. Ist dies geschehen, ändert sich der Scheitelfaktor des fließenden Stroms signifikant. Die eintretende Änderung des Scheitelfaktors wird erkannt und für die weitere Steuerung des Geräts ausgewertet.

Es hat sich herausgestellt, dass insbesondere der Übergang vom Anschnitt in die Schneid- bzw. Abtragphase kritisch sein kann. Das die Elektrode umgebende Wasser verdampft sehr schnell, so dass die im Anschnitt an der Elektrode erforderliche und tatsächlich auch an die Elektrode gelieferte Leistung schnell reduziert werden muss. Geschieht dies nicht, kann es vorkommen, dass in die beginnende Plasmaentladung zu viel Leistung geliefert wird, was zu unerwünschten Effekten führt. Andererseits muss im Anschnitt mit hoher Leistung gearbeitet werden, um eine zuverlässige Ausbildung einer Dampfblase und Plasmaentladung zu erreichen.

Davon ausgehend ist es Aufgabe der Erfindung, ein Konzept anzugeben, mit dem sich bei einer elektrochirurgischen Einrichtung ein sicherer und kontrollierter Übergang vom Anschnittbetrieb zum Schneidbetrieb erreichen lässt.

Diese Aufgabe wird mit der elektrochirurgischen Einrichtung nach Anspruch 1 gelöst:

Erfindungsgemäß wird zur Bereitstellung von HF-Leistung ein HF-Generator genutzt, der mit der Elektrode eines elektrochirurgischen Instruments verbunden oder verbindbar ist und der in seiner Leistungsabgabe steuerbar ist. Im einfachsten Fall ist er dazu ein- und ausschaltbar.

Der HF-Generator besteht mindestens aus einem Schwingkreis und einem Verstärkerbauelement, bspw. einem als nichtlineares Verstärkerbauelement genutzten elektronischen Schalter. Dieser wird zur Erregung des Schwingkreises phasenrichtig ein- und ausgeschaltet. Zur Unterbrechung der HF-Leistungserzeugung wird dieses periodische Ein- und Ausschalten des Schalters unterbrochen. Durch die Wahl des Tastverhältnisses beim Ein- und Ausschalten kann die HF-Leistung beeinflusst werden.

Die erfindungsgemäße Einrichtung umfasst eine Sensoreinrichtung. Diese erkennt das Ende der Anschnittphase und den Beginn der HF-Entladung. In diesem Fall schaltet sie den HF-Generator von Anschnittbetrieb auf Schneidbetrieb um. Der HF-Generator wird in der Anschnittphase mit hoher Stromabgabe betrieben. Im Schneidbetrieb arbeitet er mit niedrigerem Strom.

Im einfachsten Ausführungsbeispiel erfolgt das Umschalten zwischen Anschnitt- und Schneidbetrieb durch eine Änderung des Scheitelfaktors, der von dem HF-Generator bereit gestellten HF-Spannung bzw. des erzeugten HF-Stroms. Der Scheitelfaktor ist das Verhältnis des Spannungs- oder Stromspitzenwerts zu dem Spannungs- oder Stromeffektivwert. Er wird auch als Crest-Faktor bezeichnet.

Während des Anschnitts wird mit einem Scheitelfaktor zwischen 1,0 und 2,5, vorzugsweise zwischen 1,4 und 2,5, gearbeitet, während in der Schneid- bzw. Abtragbetriebsart ein Scheitelfaktor von 2,5 bis 5 angewendet wird. Erfindungsgemäß wird der Scheitelfaktor bei Erkennen der beginnenden HF-Entladung augenblicklich von dem für die Anschnittphase vorgesehenen niedrigeren Wert auf den für den Schneidebetrieb vorgesehenen höheren Wert umgeschaltet. Eine gleitende Regelung des Scheitelfaktors wird zumindest während dieses Umschaltzeitraums nicht vorgenommen, wodurch Einschwingvorgänge, die zu unerwünschten chirurgischen Effekten führen könnten, vermieden werden.

Unabhängig von der jeweiligen Größe der Scheitelfaktoren in Anschnitt und Schneidbetriebsart ist es vorteilhaft, wenn der HF-Generator in der Schneidbetriebsart einen Scheitelfaktor erzeugt, der größer ist als der Scheitelfaktor der Anschnittbetriebsart.

Das gesteuerte Umschalten der Betriebsart des HF-Generators von einer Anschnittbetriebsart in eine Schneidbetriebsart durch Umschalten des Scheitelfaktors kann bspw. durch schlagartiges Wechseln des Tastverhältnisses einer Rechteck-Modulationskurve erreicht werden, mit der die von dem HF-Generator abgegebene Schwingung moduliert ist. Mit anderen Worten, der HF-Generator wird zur Leistungsbeeinflussung periodisch ein- und ausgeschaltet. In der Anschnittbetriebsart wird mit einem hohen Verhältnis zwischen Einschaltzeit und Periodendauer gearbeitet während im Schneidbetrieb mit einem niedrigeren Verhältnis zwischen Einschaltzeit und Periodendauer gearbeitet wird.

Sofern dies nicht zu einem zu hohen Leistungseintrag in das biologische Gewebe führt, kann im Anschnitt mit einem Tastverhältnis von 1, d.h. einem Scheitelfaktor von 1,4 (Sinus) gearbeitet werden. Der HF-Generator arbeitet dabei im Dauerbetrieb. Es wird jedoch bevorzugt, auch im Anschnittbetrieb ein Tastverhältnis kleiner als 1 zu nutzen, um die in das biologische Gewebe eingebrachte Leistung zu begrenzen (z.B. 400Ws/s). Es ist dann möglich, die kurzen Austastlücken der HF-Spannung zur Leistungsreduktion zu nutzen. Außerdem kann beim Wiedereinschalten des HF-Generators jeweils zu Beginn jedes HF-Schwingungszugs an der Elektrode ein erhöhter Stromimpuls erzielt werden, der die Plasmazündung unterstützt. Dies insbesondere, wenn als HF-Generator ein Parallelschwingkreis genutzt wird, dessen Spule zu Beginn des HF-Schwingungszugs kurz mit der vollen Betriebsspannung verbunden wird.

Die den HF-Generator versorgende Einheit, bspw. ein Netzteil, kann eine geregelte oder auch eine ungeregelte Betriebsspannung zur Verfügung stellen. Der HF-Generator hat jedoch typischerweise einen (vorzugsweise positiven) Innenwiderstand, der bei niedrigem Lastwiderstand, d.h. im Anschnittbetrieb, zu einer Verminderung der an der Elektrode wirksamen Spannung führen kann.

Zur Erkennung des Übergangs von der Anschnittbetriebsart in die Schneidbetriebsart können der zu der Elektrode fließende Strom oder die an der Elektrode anliegende Spannung überwacht werden. Wird die Spannung überwacht, kann der beginnende Anschnitt anhand der an der Elektrode auftretenden Spannungserhöhung erfasst werden. Während der an der Elektrode wirksame ohmsche Widerstand im Anschnitt im Bereich von wenigen 10 Ohm bspw. 25 Ohm liegt, stellt sich im Schneidbetrieb ein deutlich höherer Widerstand von bspw. größer als 200 Ohm ein.

Es ist auch möglich, zur Erkennung der beginnenden Plasmaentladung den zu der Elektrode fließenden Strom oder die an der Elektrode anliegende Spannung spektral zu untersuchen. Ein dazu dienender Spektralanalysator kann das Eintreten der Entladung am plötzlichen Auftreten von Oberwellen erkennen, die im Anschnitt nicht vorhanden sind.

Weiter ist es möglich, einen Gleichanteil der Elektrodenspannung als Indikator für die beginnende Entladung heranzuziehen. Dieser Gleichanteil beruht nicht auf thermoionischen Effekten, sondern vorwiegend auf Feldeffekten und tritt somit auch schon bei relativ kalter Elektrode ein. Z.B. kann zumindest bei geeigneter Elektrodenform die an der Elektrode auftretende feldstärkeinduzierte Elektronenemission größer sein, als die Elektronenemission des Gewebes, das für die Plasmaentladung die Gegenelektrode bildet.

Weitere Einzelheiten vorteilhafter Ausführungsformen der Erfindung ergeben sich aus der Zeichnung sowie der nachfolgenden Beschreibung von Ausführungsbeispielen oder aus Unteransprüchen. Es zeigen:
Figur 1 eine elektrochirurgische Einrichtung, in schematischer Darstellung,
Figur 2 biologisches Gewebe mit Elektrode und Dampfblase, in schematisierter Schnittdarstellung,
Figur 3 Zeitdiagramme von Elektrodenstrom, Betriebsspannung und Modulationssignal als Zeitdiagramm,
Figur 4 den zeitlichen Verlauf des HF-Stroms während der Anschnittphase, in einem Zeitausschnitt, und
Figur 5 und 6 beispielhafte Sensoreinrichtungen, jeweils schematisch.

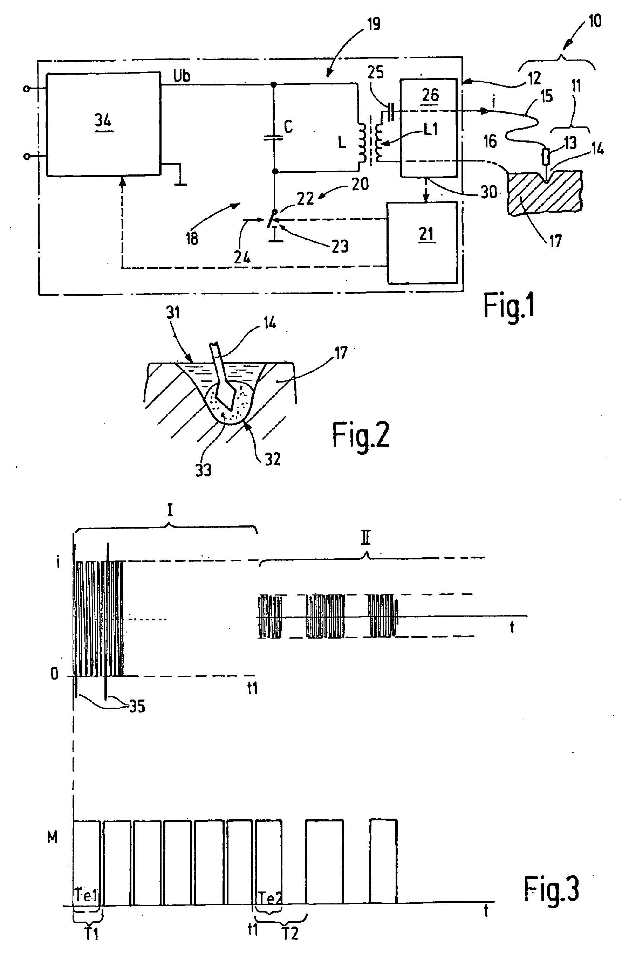

In Figur 1 ist eine elektrochirurgische Einrichtung 10 bzw. ein elektrochirurgisches System veranschaulicht, zu dem ein elektrochirurgisches Instrument 11 und ein zur Versorgung dieses Instruments 11 dienendes elektromedizinisches Gerät 12 gehören. Das Instrument 11 ist beispielsweise ein vom Operateur von Hand zu führendes Instrument mit einem vorzugsweise aus einem elektrisch isolierenden Material bestehenden Handgriff 13 und einer Elektrode 14, die an wenigstens einer Stelle einen frei liegenden metallischen Leiter aufweist. Dieser kann jede medizinisch gewünschte Form, wie bspw. Kugelform, Spatelform, Klingenform, Nadelform oder dergleichen aufweisen.

Im vorliegenden Ausführungsbeispiel ist das elektrochirurgische Instrument zumindest in den Figuren 1 und 2 als monopolares Instrument veranschaulicht. Es ist über eine speisende Leitung 15 mit dem Gerät 12 verbunden. Bei der monopolaren Ausführungsform verbindet eine weitere, als Rückleiter dienende Leitung 16 biologisches Gewebe 17, z.B. einen Patienten, an dem der chirurgische Effekt zu bewirken ist, mit dem Gerät 12. Die Leitungen 15 und 16 sind dann in getrennten Kabeln untergebracht. Die Leitung 16 kann eine galvanische oder auch kapazitive Kopplung zu dem Gewebe 17 schaffen.

In der Darstellung der Figuren 1 und 2 handelt es sich bei dem Instrument 11 um ein monopolares Instrument mit nur einer Elektrode 14. Jedoch kann es sich auch um ein bipolares Instrument handeln, dessen beide Elektroden dann von den Leitungen 15, 16 gespeist werden. Die Leitungen 15, 16 sind dann vorzugsweise in einem gemeinsamen Kabel untergebracht.

Das Gerät 12 enthält einen HF-Generator 18, der im vorliegenden Ausführungsbeispiel durch einen (Parallel-) Schwingkreis 19, ein zur Erregung dienendes elektronisches Bauelement 20 und eine Steuereinrichtung 21 gebildet ist. Das elektronische Bauelement 20 weist Verstärkereigenschaften auf. Das Bauelement 20 kann linear oder nichtlinear ausgebildet sein. Im bevorzugten Ausführungsfall handelt es sich um einen elektronischen Schalter 22, beispielsweise einen MOSFET, einen IGBT oder dergleichen. Der elektronische Schalter 22 weist eine Steuerelektrode 23 auf, die mit der Steuereinrichtung 21 verbunden ist und von dieser mit Steuersignalen versorgt wird. Der Schalter 22 bildet ein entsprechend den Steuersignalen der Steuereinrichtung 21 zu öffnenden und zu schließenden Strompfad 24 zwischen dem Schwingkreis 19 und dem Bezugspotenzial. Der Schwingkreis 19 ist außerdem an die Betriebsspannung U_{b} angeschlossen.

Der Schwingkreis 19 umfasst mindestens einen Kondensator C und eine Spule L, die zueinander parallel geschaltet sind und vorzugsweise einen Schwingkreis hoher Kreisgüte geben. Die Resonanzfrequenz des Schwingkreises 19 bestimmt die Frequenz der abgegebenen HF-Spannung von beispielsweise 350 kHz. Während die Kapazität des Kondensators C vorzugsweise zwischen 50 und 200 nF liegt, liegt die Induktivität der Spule L vorzugsweise zwischen 4µH und 1 µH. Die Spule L koppelt magnetisch mit einer Auskoppelspule L1, die über einen Auskoppelkondensator 25 und eine Sensoreinrichtung 26 mit den Leitungen 15, 16 und somit letztendlich mit der Elektrode 14 verbunden ist.

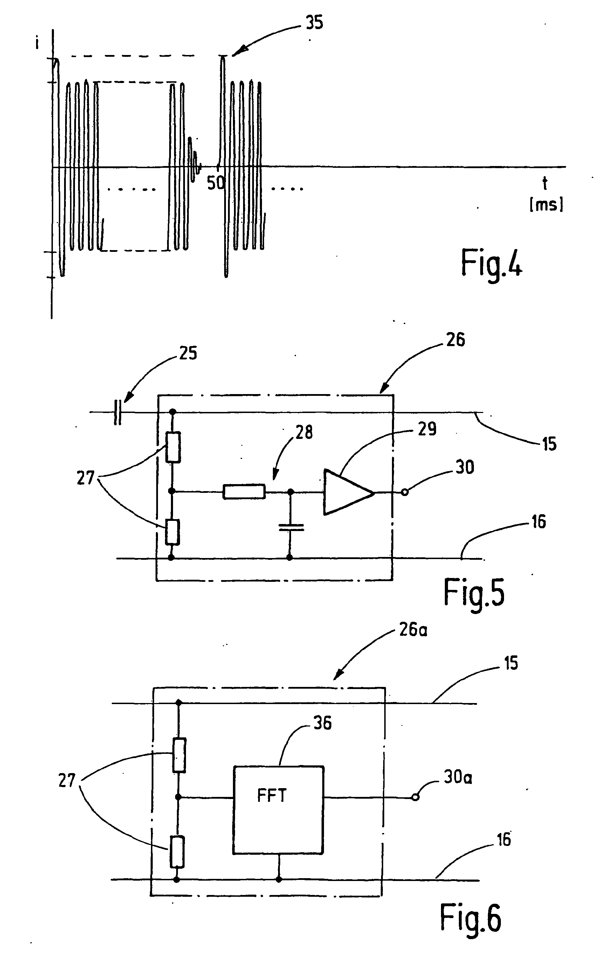

Die Sensoreinrichtung 26 dient zur Erfassung der Ausbildung einer Plasmaentladung an der Elektrode 14. Sie kann beispielsweise gemäß Fig. 5 ausgebildet sein. Ihr Eingang ist ein Spannungsteiler 27, der über den Koppelkondensator 25 an die Auskoppelspule L1 angeschlossen ist. Ein Tiefpass 28 bildet den Spannungsmittelwert, der von einem Verstärker 29 erfasst und an seinem Ausgang 30 als Signal bereitgestellt wird, das den Spannungsmittelwert kennzeichnet. Dieses Signal wird der Steuereinrichtung 21 zugeführt, die vergleicht, ob es über oder unter einem Schwellwert liegt und dementsprechend Anschnittbetrieb oder Schneid- bzw. Abtragbetrieb steuert. Anstelle des Verstärkers 29 kann auch ein Komparator vorgesehen sein.

Der Schwellwert des Komparators oder der Steuereinrichtung kann so festgelegt sein, dass ein niedriger, während der Anschnittsphase vorhandener Gleichanteil und ein hoher Gleichanteil der während des Schneidbetriebs der an der Elektrode 14 vorhandenen Spannung sicher unterschieden werden. So hat das Signal während der Anschnittsphase einen ersten Wert und während der Schneidphase einen von dem ersten Wert verschiedenen zweiten Wert. Anhand dieser Werte erfasst die Steuereinrichtung 21 den Betriebszustand an der Elektrode 14.

Zu dem Gerät 12 gehört außerdem eine hier als geregeltes Netzteil 34 ausgebildete Betriebsspannungsquelle, die den HF-Generator 18 und alle weiteren Komponenten mit Betriebspannung U_{b} versorgt. Die Betriebsspannung U_{b} beträgt beispielsweise mehrere 100 Volt, z.B. 400 Volt. Die Betriebsspannung kann z.B. zur Auswahl verschiedener chirurgischer Effekte schaltbar oder einstellbar sein. Insbesondere ist es möglich, einen Wirkpfad zwischen der Steuereinrichtung 21 und dem Netzteil 34 vorzusehen, so dass eine Leistungsregulierung und/oder die Einstellung von Eigenschaften des Netzteils 34 über Komponenten oder auch Bedienelemente der Steuereinrichtung 21 möglich ist.

Die Steuereinrichtung 21 kann einen oder mehrere elektronische Schaltkreise, insbesondere Mikrocontroller enthalten, die den Betrieb des HF-Generators 18 steuern. Die nachfolgend beschriebene Funktion des Geräts 12 lässt insbesondere die Funktion und somit auch die Programmierung eventueller Mikrocontroller in der Steuereinrichtung 21 erkennen:
Zur Funktionsbeschreibung wird auf Fig. 2 verwiesen. Diese veranschaulicht die Elektrode 14 innerhalb des Gewebes 17 umgeben von einer Flüssigkeit 31. Bei dieser handelt es sich beispielsweise um physiologische Kochsalzlösung, gegebenenfalls vermischt mit Gewebeflüssigkeit oder auch anderen Fluiden. Soll nun durch die Elektrode 14 ein chirurgischer Effekt, beispielsweise ein Schnitt oder dergleichen bewirkt werden, wird der HF-Generator 18 aktiviert. Dies kann beispielsweise durch einen nicht weiter dargestellten, vom Chirurgen zu betätigenden Schalter oder sonstige Einrichtungen erfolgen. Auf diesen Befehl hin wird der HF-Generator 18 zunächst in einer Anschnittbetriebsart betrieben. In dieser veranlasst die Steuereinrichtung 21 kurzzeitig das Schließen des Schalters 22 und danach das periodische phasenrichtige Öffnen und Schließen um den Schwingkreis 19 auf seiner Resonanzfrequenz zu erregen. Über die Auskoppelspule L1 wird dem Schwingkreis 19 HF-Leistung entzogen. Der Elektrode 14 wird somit ein hochfrequenter Strom i zugeleitet. Die Elektrode 14 steht in Kontakt mit dem Gewebe 17. Es fließt ein aus Fig. 3 ersichtlicher hochfrequenter Strom i mit einer Amplitude von mehreren, bspw. 6 bis 8, Ampere. Der dadurch erfolgende relativ hohe Leistungseintrag bewirkt in der Umgebung der Elektrode 14 eine plötzliche Verdampfung der vorhandenen Flüssigkeit und die Ausbildung einer Dampfblase 32. Dabei wird der Übergangswiderstand von der Elektrode 14 auf das Gewebe 17 sprungartig größer. In der sich bildenden Dampfblase 32 kann sich eine HF-Gasentladung bilden, die ein Plasma 33 erzeugt.

Durch die sprunghafte Widerstandsvergrößerung wird, wie Fig. 3 erkennen lässt, zum Zeitpunkt t1, bei dem sich die Dampfblase 32 bildet, der fließende Strom i sprungartig geringer. Die an der Dampfblase 32 und somit den Leitungen 15, 16 anstehende HF-Wechselspannung kann durch unterschiedliche Elektronenemission von der Elektrode 14 und des Gewebes 17 sofort einen Gleichanteil entwickeln. Dieser wird von der Sensoreinrichtung 26 erfasst. An die Steuereinrichtung 21 gelangt ein entsprechendes an dem Ausgang 30 erscheinendes Signal, das den Übergang vom Anschnittbetrieb I zum Schneidbetrieb II kennzeichnet.

Das somit von der Sensoreinrichtung 26 erzeugte Signal kennzeichnet den Übergang der Flüssigkeit 31 in die Gasoder Dampfphase und somit die Ausbildung der Dampfblase 32. Dieses Signal wird von der Steuereinrichtung 21 genutzt, den Betrieb des HF-Generators 18 von der Anschnittbetriebsart in die Schneidbetriebsart umzuschalten, die somit für Zeitpunkte größer t1 gilt. In Figur 3 ist dies veranschaulicht. Der Strom i nimmt ab und die Modulation des hochfrequenten Stroms i wird von einer ersten Modulation, die für den Zeitraum 0 bis t1 und somit für den Anschnittbetrieb gilt, auf eine zweite Modulation umgeschaltet, die für die Zeit nach dem Anschnitt, d.h. die Schneidbetriebsart gilt.

Die erste Modulation ist in Figur 3 unten links veranschaulicht. Wie ersichtlich, arbeitet der HF-Generator 18 während des Anschnittbetriebs mit keinen oder nur geringen Unterbrechungen. Somit ist sein Scheitelfaktor ungefähr 1,4 oder wenig größer als dieser, vorzugsweise aber wenigstens kleiner als 2,5. Arbeitet der HF-Generator 18 nicht in Dauerbetrieb, sondern in gepulster Betriebsart, wie es in Figur 3 unten links veranschaulicht ist, ist das Tastverhältnis T1/Te1 nahezu 1, vorzugsweise wenigstens größer als 0,7 bis 0,8. Dabei ist T1 die Periodendauer des Modulationssignals während Te1 die Einschaltdauer ist. Dies ergibt insgesamt einen Scheitelfaktor von wenig mehr als 1,4.

Bei dem Zeitpunkt t1 wird die Modulation von der ersten Betriebsart in die zweite Betriebsart umgeschaltet, in der ein größerer Scheitelfaktor vorhanden ist. Das Tastverhältnis T2/Te2 ist vorzugsweise deutlich kleiner als 0,7. Es ergibt sich der lückende HF-Wellenzug nach Figur 3, erstes oberes Diagramm, rechte Hälfte für Zeitpunkte größer t1.

Durch das sofortige Umschalten von dem niedrigen Scheitelfaktor des Anschnittbetriebs zu dem höheren Scheitelfaktor für den Schneidbetrieb bei Erkennen der Ausbildung der Dampfblase 32 wird das Einleiten übergroßer Leistungen in die sich bildende Dampfblase 32 und somit eine thermische Schädigung des Gewebes 17 und/oder der Elektrode 14 vermieden. Durch das plötzliche Umschalten der Modulation und somit des Scheitelfaktors werden außerdem die Auswirkungen langsamer Regelvorgänge und Einschwingvorgänge wie bspw. auch des Spannungsanstiegs der HF-Spannung nach dem Zeitpunkt t1 vermieden.

Figur 4 zeigt einen weiteren Aspekt, der bei der Erfindung genutzt werden kann. Wie ersichtlich, wird zumindest bei der bevorzugten Ausführungsform eine kurze periodische Aus-Tastung des HF-Generators 18 vorgenommen. Beispielsweise beträgt das Tastverhältnis T1/Te1 0,9. Zum Beispiel beträgt T1 50 ms. In der etwa 5 ms dauernden Pause zwischen zwei Wellenzügen der HF-Spannung des HF-Generators 18 schwingt die HF-Spannung aus. Zum Wiedereinschalten wird der zur Erregung dienende Schalter 22 kurz geschlossen. Durch das Schließen des Schalters 22 ergibt sich eine erste Stromspitze 35, die große Werte von bspw. bis zu 10 A_{Spitze} annehmen kann und somit zu einer erheblichen Verbesserung der Zündwilligkeit des Plasmas 33 im Anschnittbetrieb führt. Dieser Effekt kann unabhängig davon genutzt werden, wie groß die Periodendauer T1 und die Einschaltdauer Te1 gewählt sind. Auf die genannte Weise ergibt sich so für den Strom i während des Anschnittbetriebs ein fast durchgehender HF-Wellenzug mit periodischen Stromspitzen 35, die z.B. durch periodisch wiederkehrende Einschwingvorgänge des Schwingkreises 19 verursacht werden.

Alle Ausführungsformen der erfindungsgemäßen Einrichtung 10 erfordern eine zuverlässige Erkennung der Ausbildung der Dampfblase 32 und des Plasmas 33. Alternativ zu der oben beschriebenen Erfassung des Gleichanteils der HF-Spannung an der Elektrode 14 können dazu auch andere charakteristische elektrische Größen ausgewertet werden. Z.B. hat die an der Elektrode 14 anliegende HF-Spannung infolge des Innenwiderstands des HF-Generators während des Anschnittbetriebs einen geringeren Wert als während des Schneidbetriebs. Die Sensoreinrichtung 26 kann eine Schwellwertschaltung sein. Das Ausgangssignal derselben zeigt an, ob der Betrag der HF-Spannung größer oder kleiner als ein geeignet festgelegter Grenzwert ist. Das Ausgangssignal wird wiederum zu der Steuereinrichtung 21 geleitet.

Figur 6 veranschaulicht eine weitere alternative Sensoreinrichtung 26a. In der Sensoreinrichtung 26a ist ein an den Spannungsteiler 27 angeschlossenes Frequenzanalysemodul 36 vorgesehen, das das Spektrum der hochfrequenten zwischen den Leitungen 15, 16 anliegenden HF-Spannung erfasst. Die anstehende Spannung wird von dem Frequenzanalysemodul 36 analysiert. Dies kann z.B. mittels schneller Fourier-Transformation erfolgen, die hier von einem Mikrocontroller ausgeführt werden kann. Das Frequenzanalysemodul 36 wertet dann das Spektrum z.B. auf Vorhandensein charakteristischer Spektrallinien oder Spektralanteile aus und gibt an dem Ausgang 30a ein Signal ab, wenn anhand des Spektrums die Ausbildung der Dampfblase 32 erkannt wird. Die charakteristische Veränderung des Spektrums ergibt sich aus der Änderung der Charakteristik des Übergangswiderstands von der Elektrode 14 zu dem Gewebe 17. Solange die Elektrode 14 über Natriumchloridlösung (NaCl) mit dem Gewebe 17 kontaktiert ist, ist der Übergangswiderstand näherungsweise linear. Sobald das Plasma 33 zündet, ist er jedoch hochgradig nichtlinear, wodurch charakteristische Oberwellen erzeugt werden.

Die Sensoreinrichtung 26, 26a wird zur Plasmaerkennung zur sofortigen Einleitung des Schneidbetriebs genutzt. Sollte die Sensoreinrichtung 26, 26a hingegen das Verlöschen des Plasmas 33 durch Änderung des entsprechenden Signals an dem Ausgang 33 erkennen, kann die Steuereinrichtung 21 entsprechend reagieren und das Gerät 12 und seinen HF-Generator 18 wiederum in die Anschnittbetriebsart I zurück überführen. Damit wird es möglich, auch mit ständig wiederkehrenden Löschereignissen mit immer wiederkehrender Anschnittbetriebsart zu arbeiten, bspw. um Gewebeabtragungen durchzuführen. Dies gilt für alle Arten der beschriebenen Sensoreinrichtungen und Modulationsarten.

Zur Erzielung eines sicheren Übergangs vom Zünden eines Plasmas 33 im Anschnittbetrieb in das Brennen des Plasmas 33 während des Schneidbetriebs, wird vorgesehen, ein elektromedizinisches Gerät 12 zur Versorgung eines Instruments 11 mit elektrischer Leistung mit einer Zünderkennung auszurüsten, die durch eine Sensoreinrichtung 26 gebildet wird. Diese Zünderkennung schaltet den in dem Gerät 12 vorhandenen HF-Generator 18 von einer Anschnittbetriebsart I in eine Schneidbetriebsart II um, sobald das Zünden erkannt wird. Das Umschalten erfolgt durch Umschalten einer HF-Modulation vorzugsweise von einem niedrigen Scheitelfaktor von weniger als 2,5 in der Anschnittbetriebsart zu einem hohen Scheitelfaktor von größer als 2,5 in der Schneidbetriebsart.

Bezugszeichenliste:
- 10: elektrochirurgische Einrichtung/System
- 11: elektrochirurgisches Instrument
- 12: elektromedizinisches Gerät
- 13: Handgriff
- 14: Elektrode
- 15: speisende Leitung
- 16: rückführende Leitung
- 17: Gewebe
- 18: HF-Generator
- 19: Schwingkreis/Parallelschwingkreis
- 20: elektronisches Bauelement
- 21: Steuereinrichtung
- 22: Schalter
- 23: Steuerelektrode
- 24: Strompfad
- U_{b}: Betriebsspannung
- C: Kondensator des Schwingkreises 19
- L: Spule des Schwingkreises 19
- L1: Auskoppelspule
- 25: Koppelkondensator
- 26, 26a: Sensoreinrichtung
- 27: Spannungsteiler
- 28: Tiefpass
- 29: Verstärker
- 30, 30a: Ausgang
- 31: Flüssigkeit
- 32: Dampfblase
- 33: Plasma
- 34: Netzteil, Betriebsspannungsquelle
- 35: Stromspitze
- 36: Frequenzanalysemodul
- I: Anschnittbetriebsart
- II: Schneidbetriebsart

## Patentansprüche

1. Elektrochirurgische Einrichtung (10) mit einem eine Elektrode (14) enthaltenden Instrument (11), sowie mit einem Gerät (12) zur Versorgung des Instruments (11) mit elektrischer Leistung, wobei das Gerät (12):
einen steuerbaren HF-Generator (18) zur Erzeugung von HF-Leistung in einer Anschnittbetriebsart (I) während einer Anschnittphase, bei der die in einem feuchten Gewebemilieu platzierte Elektrode (14) umgebende Flüssigkeit (31) verdampft, sowie in einer Schneidbetriebsart (II) während einer Schneidphase, während derer an der Elektrode (14) eine HF-Entladung aufrechterhalten wird, und
eine Sensoreinrichtung (26, 26a) zur Erkennung einer eingetretenen Verdampfung und beginnenden HF-Entladung aufweist, **dadurch gekennzeichnet,**
**dass** die Sensoreinrichtung (26, 26a), wenn sie die beginnende HF-Entladung erfasst, den HF-Generator (18) von der Anschnittbetriebsart (I) in die Schneidbetriebsart (II) überführt,
**dass** der HF-Generator (18) in der Anschnittbetriebsart (I) einen Scheitelfaktor zwischen 1,0 und 2,5 erzeugt und
**dass** der HF-Generator (18) in der Schneidbetriebsart (II) einen Scheitelfaktor erzeugt, der größer ist als der Scheitelfaktor der Anschnittbetriebsart (I).

2. Elektrochirurgische Einrichtung nach Anspruch 1, **dadurch gekennzeichnet, dass** der HF-Generator (18) die HF-Leistung als Sinusschwingung bereitstellt, wobei der Scheitelfaktor in der Anschnittbetriebsart (I) zwischen 1,4 und 2,5 beträgt.

3. Elektrochirurgische Einrichtung nach Anspruch 1, **dadurch gekennzeichnet, dass** der HF-Generator (18) in der Anschnittbetriebsart (I) wiederholt aussetzend arbeitet.

4. Elektrochirurgische Einrichtung nach Anspruch 3, **dadurch gekennzeichnet, dass** der HF-Generator (18) einen Schwingkreis (19) und einen im Schaltbetrieb arbeitenden, mit dem Schwingkreis (19) verbundenen elektronischen Schalter (22) aufweist, um den Schwingkreis (19) wiederholt mit der vollen Betriebsspannung zu beaufschlagen.

5. Elektrochirurgische Einrichtung nach Anspruch 1, **dadurch gekennzeichnet, dass** die Sensoreinrichtung (26, 26a) eine Amplitudenerfassungseinrichtung, eine Gleichanteilserfassungseinrichtung oder eine Oberwellenerfassungseinrichtung aufweist.

6. Elektrochirurgische Einrichtung nach Anspruch 1, **dadurch gekennzeichnet, dass** der HF-Generator (18) während des Betriebs des HF-Generators (18) in der Anschnittsbetriebsart (I) eine niedrigere Spannung liefert als während des Betriebs des HF-Generators (18) in der Schneidbetriebsart (II).

## Claims

1. Electrosurgical arrangement (10) with an instrument (11) containing an electrode (14) and with a device (12) for supplying the instrument (11) with electrical power, wherein the device (12):
has a controllable HF-generator (18) for generating HF-power in an incising mode of operation (I) during an incising phase, in which the liquid (31) surrounding [the] electrode (14) positioned in a moist tissue environment evaporates, and also in a cutting mode of operation (II) during a cutting phase, during which an HF-discharge is maintained at the electrode (14), and
has a sensor arrangement (26, 26a) to detect an evaporation has occurred and HF-discharge has begun, **characterised in that**
when it detects that the HF-discharge has begun, the sensor arrangement (26, 26a) shifts the HF-generator (18) from the incising mode of operation (I) to the cutting mode of operation (II),
that in the incising mode of operation (I) the HF-generator (18) generates a peak factor between 1.0 and 2.5, and
that in the cutting mode of operation (II) the HF-generator (18) generates a peak factor, which is higher than the peak factor of the incising mode of operation (I).

2. Electrosurgical arrangement according to claim 1, **characterised in that** the HF-generator (18) provides the HF-power as sinusoidal oscillation, wherein the peak factor in the incising mode of operation (I) amounts to between 1.4 and 2.5.

3. Electrosurgical arrangement according to claim 1, **characterised in that** the HF-generator (18) repeatedly operates intermittently in the incising mode of operation (I).

4. Electrosurgical arrangement according to claim 3, **characterised in that** the HF-generator (18) has an oscillatory circuit (19) and an electronic switch (22), which operates in switching operation and is connected to the oscillatory circuit (19) in order to repeatedly supply the oscillatory circuit (19) with the full operating voltage.

5. Electrosurgical arrangement according to claim 1, **characterised in that** the sensor arrangement (26, 26a) has an amplitude detection device, a d.c. component detection device or a harmonic detection device.

6. Electrosurgical arrangement according to claim 1, **characterised in that** during operation of the HF-generator (18) in the incising mode of operation (I) the HF-generator (18) supplies a lower voltage than during the operation of the HF-generator (18) in the cutting mode of operation (II).

## Revendications

1. Dispositif électrochirurgical (10) comprenant un instrument (11), lequel contient une électrode (14), ainsi qu'un appareil (12) pour alimenter l'instrument (11) en puissance électrique, lequel appareil (12) comprend :
un générateur HF commandable (18) pour générer une puissance HF dans un mode de fonctionnement en incision (I) pendant une phase d'incision, où l'électrode (14) placée dans un milieu de tissu humide vaporise le liquide environnant (31), ainsi que dans un mode de fonctionnement en coupe (II) pendant une phase de coupe, où une décharge HF est maintenue à 'l'électrode (14), et
un dispositif capteur (26, 26a) pour détecter une vaporisation survenue et une décharge HF commençante, **caractérisé**
**en ce que** le dispositif capteur (26, 26a), lorsqu'il détecte la décharge HF commençante, fait passer le générateur HF (18) du mode de fonctionnement en incision (I) au mode de fonctionnement en coupe (II),
**en ce que** le générateur HF (18) génère, dans le mode de fonctionnement en incision (I), un facteur de crête compris entre 1,0 et 2,5 et
**en ce que** le générateur HF (18) génère, dans le mode de fonctionnement en coupe (II), un facteur de crête supérieur au facteur de crête du mode de fonctionnement en incision (I).

2. Dispositif électrochirurgical selon la revendication 1, **caractérisé en ce que** le générateur HF (18) fournit la puissance HF sous la forme d'une oscillation sinusoïdale, le facteur de crête dans le mode de fonctionnement en incision (I) étant compris entre 1,4 et 2,5.

3. Dispositif électrochirurgical selon la revendication 1, **caractérisé en ce que** le générateur HF (18) présente un fonctionnement intermittent répété dans le mode de fonctionnement en incision (I).

4. Dispositif électrochirurgical selon la revendication 3, **caractérisé en ce que** le générateur HF (18) comprend un circuit résonnant (19) et un commutateur électronique (22) fonctionnant en tout ou rien et relié au circuit résonant (19) pour soumettre le circuit résonant (19) de manière répétée à la tension de fonctionnement complète.

5. Dispositif électrochirurgical selon la revendication 1, **caractérisé en ce que** le dispositif capteur (26, 26a) comprend un dispositif de détection d'amplitude, un dispositif de détection de composante continue ou un dispositif de détection d'ondes harmoniques.

6. Dispositif électrochirurgical selon la revendication 1, **caractérisé en ce que** le générateur HF (18) délivre une tension plus basse pendant son fonctionnement dans le mode de fonctionnement en incision (I) que pendant son fonctionnement dans le mode de fonctionnement en coupe (II).
